# EUROPEAN PATENT SPECIFICATION

(11) **EP 4 260 814 B1**
(45) Date of publication and mention of the grant of the patent: **06.05.2026**
(21) Application number: 21782867.2
(22) Date of filing: 09.07.2021
(51) Int. Cl.: A61B 17/00

(54) **PUNCTURABLE INTERATRIAL SEPTUM OCCLUDER**
DURCHSTECHBARER INTERATRIALER SEPTUMVERSCHLUSS
DISPOSITIF D'OCCLUSION DE SEPTUM INTERAURICULAIRE POUVANT ÊTRE PERFORÉ

(30) Priority: 11.12.2020 CN 202011444360
(43) Date of publication of application: 18.10.2023
(73) Proprietor: Shanghai HanYu Medical Technology Co., Ltd, Shanghai 201109 (CN)
(72) Inventor: PAN, Wenzhi, Shanghai 201109 (CN); ZHOU, Daxin, Shanghai 201109 (CN); GE, Junbo, Shanghai 201109 (CN)
(74) Representative: Santarelli
(86) International application number: PCT/CN2021/105364
(87) International publication number: WO 2022/121300

(56) References cited:
- CN-A- 110 236 606
- CN-A- 111 956 275
- CN-A- 111 956 275
- CN-U- 205 072 922
- CN-U- 205 625 989
- CN-U- 207 236 817
- CN-U- 211 583 282
- CN-U- 211 583 282
- US-A1- 2005 065 547
- US-A1- 2011 054 519
- US-A1- 2014 257 360
- US-A1- 2017 273 790

## Description

### Technical Field

The present invention relates to the field of medical device technologies, and more particularly, to a puncturable atrial septal occluder.

### Background of the Invention

An atrial septal defect is a common congenital heart disease. A current mainstream treatment is to use an occluder to perform a percutaneous (a femoral vein) atrial septal occlusion. In this operation, the occluder is one of the most important medical device.

The occluder currently in use is of an "H"-shaped double-disc and one-waist nickel-titanium alloy woven structure, and the safety and effectiveness of the structure have been widely clinically proven. Most of patients undergoing atrial septal defect occlusion are young patients, and there is a high probability of complications such as atrial fibrillation and mitral regurgitation in a later stage. These complicated diseases can be treated by radiofrequency ablation, left atrial appendage occlusion, mitral valve clamping, etc., which puncture an atrial septum again into a left atrium via a femoral vein for a minimally invasive interventional surgery.

However, due to a large volume and a dense mesh of the occluder in a prior art, the entire atrial septum is almost filled up after implantation in the human body. A double-disc, four-layer, dense-mesh and small-mesh (φ2mm) design makes it impossible to puncture the atrial septum to deliver a sheathing canal (φ3-9mm) into the left atrium. As a result, the patient loses an interventional channel of the atrial septum, and also loses an opportunity of minimally invasive interventional treatments for the atrial fibrillation, the mitral valve regurgitation and other diseases.

For example, an occluder a disclosed in the patent CN2524710Y (see FIG. 1) has a double-disc and one-waist structure, which is not provided with a central through hole, resulting in a puncture sheathing canal being unable to pass through the occluder a at all in later need to puncture.

An occluder b disclosed in the patent CN101234042A (see FIG. 2) also has a double-disc and one-waist structure. Similarly, the occluder b is not provided with a central through hole, and a memory alloy wire is added in a waist. When the atrial septal puncture is needed later, the occluder b cannot be passed through at all.

On the basis of the forgoing patent technology, the applicant designed an occluder c disclosed in the patent CN206228378U (see FIG. 3). The occluder has a small hole at the center thereof, which is designed to leave a small through hole for an incompletely enclosed atrial septum defect so that blood can still flow through the small through hole to relieve the patient's condition after the occurrence of pulmonary hypertension in a later stage. The occluder does not completely enclose a defective atrial septum, and a blood flow can still pass through the through hole, which causes a blocking effect to descend. The size of the central through hole is too small (φ5-8mm, a waist has a much greater diameter than the through hole), which makes it difficult for a delivery sheathing canal (φ3-9mm) to pass through the defect. Further, the through hole itself has a certain length and the delivery sheathing canal is curved, which further makes it difficult for the delivery sheathing canal to pass through the through hole. The occluder may even be driven by a hanging wall, causing more serious consequences.

Patent document US20170273790A1 discloses a blood flow regulator device, intended for implantation in the atrial septum of the heart. The primary function of the device is to regulate blood flow and manage pressure differentials within the heart, which can be beneficial in treating conditions such as heart failure or pulmonary hypertension. The device features a proximal disc, a distal disc, and a neck section that forms a cavity to maintain the desired blood flow rate. The distal disc includes loops of wire that ensure a smooth surface to promote endothelialization and reduce the risk of emboli formation.

To this end, it is very necessary to design an atrial septal occluder that can safely and easily puncture and keep the atrial septal occlusion safe and effective.

### Summary of the Invention

The invention is set out in the appended set of claims. The present invention breaks through the problems of the prior art, and designs a safe and effective atrial septal occluder that can support an apparatus to be punctured at the center thereof and be delivered with a sheathing canal. Based on the premise of guaranteeing the atrial septal occluder to be safe and effective, the atrial septal occluder reserves a channel for an interval puncture intervention surgery, giving a patient the opportunity to perform a minimally invasive interventional surgery across an atrial septum.

In order to achieve the forgoing objective, the present invention designs a puncturable atrial septal occluder, including a stent made by heat setting after integrally weaving a wire material with a memory property. The stent consists of a left disc, a right disc, and a waist connected. The occluder further includes a central through hole that penetrates the stent, and a notch formed at centers of the left disc and the right disc after the penetration. The central through hole has a diameter close to an inner diameter of the waist to the greatest extent to ensure that a puncture sheathing canal can safely and easily pass through the occluder into a left atrium. A baffle membrane is provided at the central through hole to block the central through hole from the inside thereof to ensure that a blood flow is blocked, thereby ensuring that an atrial septal occlusion is safe and effective. A notch at a center of the right disc is provided with a plurality of connecting wires. One end of each of the plurality of connecting wires is converged and connected to a delivery component, and the other end of each of the plurality of the connecting wires is radially connected to the right disc, thereby realizing connection between the occluder and a delivery system. Therefore, the occluder can be pulled in or retracted into the delivery sheathing canal.

Further, the occluder has morphological memory, with two morphologies of contraction and expansion, where the contraction morphology is a strip-shaped delivering morphology in which the occluder is compressed into the sheath canal, and the expansion morphology is an "H"-shaped two-disc one-waist working morphology after free expansion. Since the occluder has a morphological memory feature, the occluder can be allowed to automatically restore to a working morphology from a delivering morphology.

Further, the baffle membrane has a sheet shape and is made of a polymer material or a degradable material. The sheet-shaped morphology of the baffle membrane has the same cross sectional shape as the central through hole. The baffle membrane is arranged in the central through hole to block the blood flow. However, since the baffle membrane is relatively thin and fragile, easy to pierce and expand, and is convenient to puncture, when the puncture is needed, the sheath canal can easily penetrate the baffle membrane through the central through hole, and then pierce the baffle membrane into a left atrium for interventional treatment.

Further, the left disc, the right disc, and the waist are of an integral mesh-shaped structure. The integral mesh-shaped structure is made by heat setting after integrally weaving a wire material. The wire material is a memory alloy material, and preferably a nickel-titanium alloy material. The memory alloy material has a memory function so that the manufactured occluder stent has the morphological memory, and hence can automatically restore to the working morphology from the delivering morphology. The mesh-shaped weaving of the waist is conducive to strengthening its support force and the restoration of memory morphology.

Further, both the left and the right discs are of a double-layer mesh-shaped structure. The double-layer structure helps strengthen the support force of the disc-shaped structure, and the mesh-shaped weaving is beneficial to the restoration of the memory morphology of the structure.

Edges of the notches at the centers of the left disc and the right disc are both densely woven. A weaving density of a reinforced weaving area is 2-5 times as much as that of other common areas to strengthen the ability of the occluder to restore the memory morphology.

Further, a connecting position of the connecting wire on the right disc is an outer layer of the right disc, and a connecting method is an integral weaving and shaping connection or a split-type physical connection.

The integral weaving and shaping connection ensures that connection between the connecting wire and the right disc is smooth and does not cause wire loss. However, due to the integral connection, a number of the connecting wires and the connecting position cannot be changed.

Relative to the integral weaving and shaping connection, the split-type physical connection is that the connecting wire is made into an independent structure, and then connected to the right disc. As the connection method, welding, stitching, weaving access or other connection methods can be selected. To ensure the firmness and flatness of the connection, the weaving access method is mostly adopted in a specific implementation. The split-type physical connection can make adaptive changes to the number of the connecting wires, connecting positions, etc. It is extremely easy to increase or reduce the connecting wires, thereby increasing the adaptability of the occluder.

Further, when the connecting wire is physically connected in the split-type physical connection, the connecting wire is of the independent structure and made of metal or a polymer wire material or a degradable polymer material, so that the connecting wire has the morphology memory. The independent structure of the connecting wire allows the number of the connecting wires and the connection position between the connecting wires and the right disc to be adjusted adaptively, quickly and easily.

Further, the connecting wires are distributed symmetrically relative to the delivery component, and a gap through which the puncture sheath canal passes is provided between the adjacent connecting wires, to ensure that the sheath canal can smoothly pass through the atrial septal occluder and enter the left atrium to finish the puncture.

Further, the number of the connecting wires is at most 12, and at least 3. Too many connecting wires reduce the gap between adjacent connecting wires, resulting in the puncture sheath canal being unable to penetrate from the right disc into the central through hole or enter the left atrium. However, few connecting wires affect the delivery of the atrial septal occluder and the anchoring of the right disc in a right atrium. Therefore, with a lot of experiments, the preferred number of the connecting wire is 3-12.

Further, the delivery component is configured as a wire-clamped steel sleeve or a condensed ball. One end of the delivery component is connected to the connecting wire, and the other end of the delivery component is a free end. The free end is provided with a connecting port and connected to the delivery system.

Compared with the prior art, the present invention maintains the structure and working principle of the traditional occluder, and ensures the safety and effectiveness of the occluder. A fully enclosed sheet-shaped baffle membrane is arranged in the central through hole to block blood from flowing through the occluder while utilizing the fragile nature of the baffle membrane to make the baffle membrane easy to be punctured and expanded. Therefore, the sheath canal can easily puncture and be delivered from the central through hole of the occluder, thereby reserving a channel for a subsequent atrial septal puncture interventional operation.

### Brief description of the Drawings

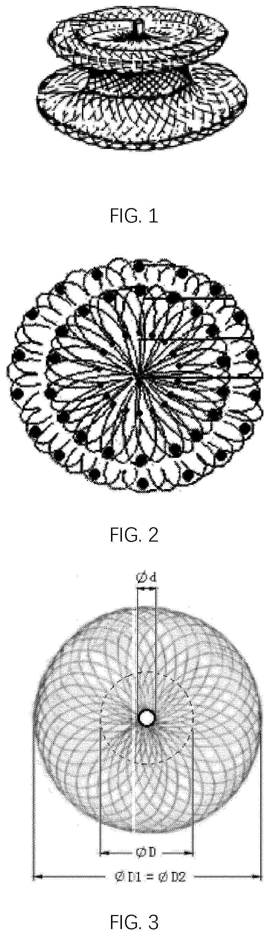

FIG. 1 shows an atrial septal occluder a in a prior art.
FIG. 2 shows an atrial septal occluder b in the prior art.
FIG. 3 shows an atrial septal occluder c in the prior art.
FIG. 4 shows a top view of a puncturable atrial septal occluder according to a specific embodiment of the invention (a baffle membrane is not shown).
FIG. 5 shows a cross-sectional view of a puncturable atrial septal occluder in an expansion morphology (left and right discs are asymmetrical) according to a specific embodiment of the invention.
FIG. 6 shows a comparison diagram of a puncturable atrial septal occluder with a central encryption weaving design according to the invention (left) and a non-central encryption weaving design**.**
Fig. 7 shows a cross-sectional view of a puncturable atrial septal occluder in an expansion configuration (left and right discs are symmetrical) according to a specific embodiment of the invention.
FIG. 8 shows a cross-sectional view of a puncturable atrial septal occluder in a contraction configuration (left and right discs are asymmetrical) according to a specific embodiment of the invention.

Where, 1: left disc, 2: right disc, 3: waist, 4: central through hole, 5: baffle membrane, 6: connecting wire, 7: delivery component.

### Detailed Description of Embodiments

The present invention is further described below in conjunction with the accompanying drawings, but is not taken as a limitation to the present invention.

It should be noted that the following detailed descriptions are all illustrative and are intended to provide further descriptions of the present invention. Unless otherwise specified, all technical and scientific terms used herein have the same meaning as commonly understood by the person skilled in the art to which the present invention belongs.

It should be noted that the terms used here are only for describing specific embodiments, and are not intended to limit the exemplary embodiments according to the present invention.

In the present invention, orientation or positional relationships indicated by terms such as "upper", "lower", "front", "rear", "vertical", "horizontal", "side", "bottom", "top", etc. are based on the orientation or positional relationship shown in the drawings. These relation terms are only determined to facilitate the description of the structural relationship of each component or element of the present invention, and do not specifically refer to any component or element in the present invention, and cannot be understood as a limitation to the present invention.

In the present invention, terms such as "fixedly connected", "interconnected", "connected", etc. should be understood in a broad sense, indicating that "fixedly connected", "interconnected", "connected", etc. can be a fixed connection, an integral connection or a detachable connection or can be directly connected, or be indirectly connected via an intermediate medium. For the relevant scientific research or the person skilled in the art, the specific meanings of the forgoing terms in the present invention can be determined according to the specific situation, and should not be understood as a limitation of the present invention.

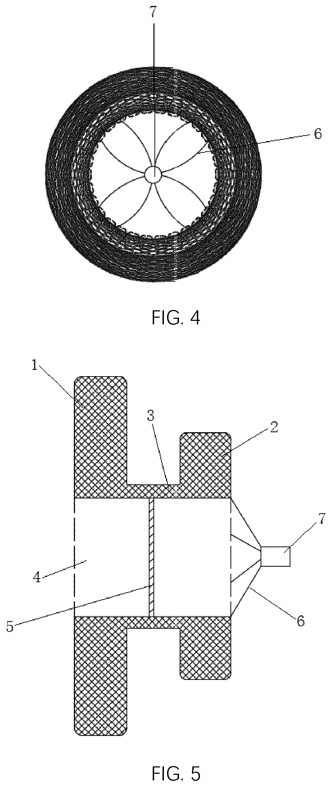

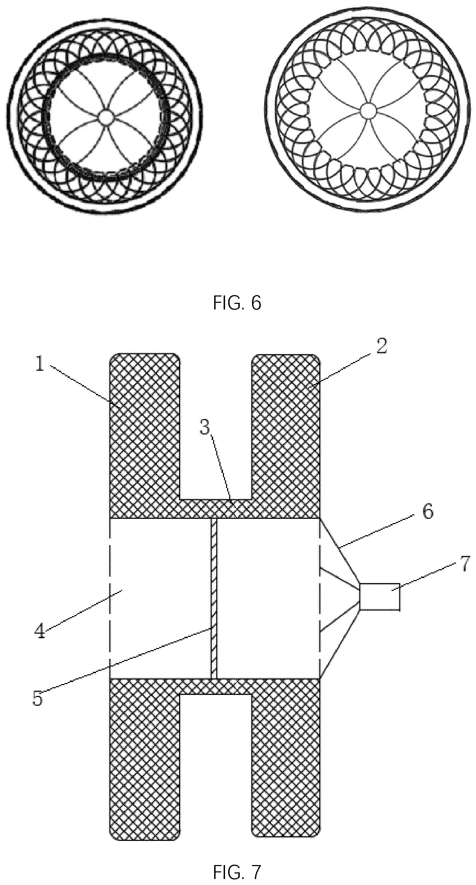

In a specific embodiment, terms such as "left" and "right" correspond to left and right atrium directions in which a corresponding occluder is implanted into the heart, respectively. Left and right discs can be symmetrical discs or asymmetrical discs and can be selected according to the condition of atrial septal defect, see FIG. 5 and FIG. 7.

Referring to FIGS. 4 to 7, a puncturable atrial septal occluder is designed in a specific embodiment of the invention, and includes a left disc 1, a right disc 2, a waist 3, and a central through hole 4. The left disc 1 and the right disc 2 are connected with the waist 3. The central through hole 4 penetrates the left disc 1, the waist 3, and right disc 2 in sequence. A notch is formed at each of the centers of the left disc 1 and right disc 2. The central through hole has a diameter close to the inner diameter of the waist to the greatest extent, to ensure that a puncture sheath canal can safely and easily pass through the occluder and enter a left atrium. One to four layers of baffle membranes 5 are sewn in the central through hole 4 to completely block the central through hole 4 from the inside thereof, thereby blocking blood from circulating. The baffle membrane 5 can be made of any one of polymer materials such as a polyester tectorial membrane, a polyurethane tectorial membrane, a polytetrafluoroethylene tectorial membrane, etc., or made of a degradable material. The sheet-shape morphology of the baffle membrane has the same cross-sectional shape as the central through hole 4. Referring to FIG. 5, the central through hole 4 has a circular cross section in this embodiment, so the baffle membrane is also circular.

Referring to FIGS. 4 and 5, the notch at the center of the right disc 2 is provided with a connecting wire 6. One end of the connecting wire 6 is converged above the notch at the center of the right disc 2, and is connected to a delivery component 7. The other end of the connecting wire 6 is radially connected to the right disc 2.

Preferably, the connecting position of the connecting wire 6 and the right disc 2 is an outer layer of the right disc, and a connecting method is an integral weaving and shaping connection or a split-type physical connection.

When the integral weaving and shaping connection is used for shaping, the connecting wire has the same material as a main body occluder.

When the split-type physical connection is used, the connecting wire is of an independent structure and can be made of metal or a polymer wire material or a degradable polymer material. Afterwards, the connecting wire is connected to the outer layer of the right disc by welding, weaving, or stitching. The outer layer refers to an outer surface layer in a double-layer structure of the right disc.

The number of the connecting wires 6 is at most 12, and at least 3, and the connecting wires are arranged to be symmetrically and rotatably distributed relative to the delivery component 7. The connecting wire and the delivery component realize connection between the main body of the occluder and a delivery system, so that the occluder can be pulled in or retracted into a delivery sheath canal.

For the number of the connecting wires, too many connecting wires reduce the gap between adjacent connecting wires, resulting in the puncture sheath canal being unable to penetrate from the right disc into the central through hole or enter the left atrium. However, few connecting wires affect the delivery of the atrial septal occluder and the anchoring of the right disc in a right atrium. Therefore, with a lot of experiments, the preferred number of the connecting wires is 3 to 12.

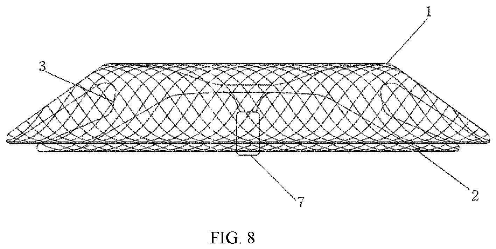

The left disc 1, the right disc 2, and the waist 3 are of an integral mesh-shaped structure. The integral mesh-shaped structure is made by heat setting after integrally weaving the wire material. The wire material is a memory alloy material, and preferably a nickel-titanium alloy, so that the occluder has morphological memory and hence can be allowed to be converted between a contraction morphology and an expansion morphology. Referring to FIG. 5, FIG. 7 and FIG. 8, the contraction morphology is a strip-shaped delivering morphology in which the occluder is compressed into the sheath canal. The expansion morphology is an "H"-shaped double-disc one-waist working morphology after free expansion. Since the occluder has the feature of morphology memory, the occluder can automatically restore to the working morphology from the delivering morphology, and the mesh-shaped weaving of the waist is beneficial to strengthening its support force and restoring memory morphology.

Both the left disc 1 and the right disc 2 are of a double-layer mesh-shaped structure. The double-layer structure helps strengthen the support force of the disc-shaped structure, and the mesh-shaped weaving is beneficial to the restoration of the memory morphology of the structure.

Densified weaving is selectively used for edges of the notches at the centers of the left disc 1 and the right disc 2. Referring to FIG. 6, the weaving density of a reinforced weaving area is 2-5 times as much as that of other common areas to strengthen the ability of the occluder to restore the memory morphology.

The delivery component 7 is configured as a wire-clamped steel sleeve or a condensed ball. One end of the delivery component 7 is connected to the connecting wire, and the other end of the delivery component 7 is a free end. The free end is provided with a connecting port and connected to the delivery system. Thus, the occluder can be pulled in or pushed out of the delivery sheath canal as a whole.

In a specific embodiment, the waist of the occluder designed in the present invention is a short waist with a length of only 2-8mm. The central through hole is circular, so that the selected baffle membrane is also a circular sheet-shaped membrane to ensure that blood is blocked from circulating.

Further, for another special feature, the central through hole designed in the present invention has a much larger diameter than a mesh (2mm) in the prior art. The diameter is close to the inner diameter of the waist to the greatest extent. In a specific embodiment, the diameter of the central through hole has a size between 8 mm and 46 mm, so as to ensure that the puncture sheath canal can safely and easily pass through the occluder and enter the left atrium.

The occluder designed in the present invention has many models, for example, the specifications are divided according to different diameters of the through hole. When in use, applicable models can be selected according to the condition of the atrial septal defect. In addition, the large through hole of the present invention significantly reduces the difficulty of a reserved puncture channel during puncture, and enhances the success rate of a subsequent atrial septal puncture interventional operation.

In a specific embodiment, the present invention maintains the traditional double-disc and one-waist structure and working principle to ensure the safety and effectiveness of the occluder. In addition, the large through hole with baffle membrane arranged at the center of the occluder can block the blood from flowing through the occluder. In addition, due to the fragility of the baffle membrane, the baffle membrane is easy to puncture and expand, making it very easy to puncture from the center of the occluder and deliver the sheathing canal. The present invention not only achieves the safe and effective treatment of the atrial septal defect, but also reserves the channel for a subsequent interventional operation via atrial septal puncture, thereby reserving an opportunity for the patient to perform a minimally invasive interventional operation across the atrial septal.

The forgoing is only the preferred embodiments of the present invention.

## Claims

1. A puncturable atrial septal occluder, comprising a stent made by heat setting after integrally braiding a wire material with a memory property, the stent consisting of a left disc (1), a right disc (2), and a waist (3) that are connected, the occluder further comprising a central through hole (4) that penetrates the stent, and a notch formed at the centers of the left disc (1) and the right disc (2), wherein the left disc (1), right disc (2), and the waist (3) are of an integral mesh-shaped structure with a wire material as a memory alloy material, the left disc (1) and the right disc (2) both have a double-layer mesh-shaped structure, edges of notches at centers of the left disc (1) and the right disc (2) are both densely woven, a weaving density of a reinforced weaving area is 2-5 times as much as that of other common areas, the central through hole (4) has a diameter close to an inner diameter of the waist (3) to the greatest extent and is configured such that a puncture sheath is capable of passing therethrough, a baffle membrane (5) is provided inside the central through hole (4) to block the central through hole (4) from the inside thereof; a notch at the center of the right disc (2) is provided with a plurality of connecting wires (6), one end of each of the plurality of connecting wires (6) is converged and connected to a delivery component (7), and the other end of each of the plurality of the connecting wires (6) is radially connected to the right disc (2) with regards to the center of the right disc (2), **characterized in that** the plurality of the connecting wires (6) are distributed rotatably and symmetrically with respect to a delivery component (7) and the central through hole (4), and a gap through which the puncture sheathing canal can pass is provided between the adjacent connecting wires, a number of the connecting wires (6) is at most 12, and at least 3, the delivery component (7) is configured as a wire-clamping steel sleeve or a condensed ball, one end of the delivery component (7) is connected to the connecting wire, the other end of the delivery component (7) is a free end, and the free end is provided with a connecting port, and connected to a delivery system, so that the puncturable atrial septal occluder is capable of being pulled into or pushed out of a delivery sheath.

2. The puncturable atrial septal occluder according to claim 1, wherein the occluder has morphological memory, with two morphologies of contraction and expansion.

3. The puncturable atrial septal occluder according to claim 1, wherein the baffle membrane (5) has a sheet shape and is made of a polymer material or a degradable material, and the sheet-shaped morphology of the baffle membrane has a cross sectional shape being the same as the central through hole (4).

4. The puncturable atrial septal occluder according to claim 1, wherein a connecting position of the connecting wire (6) on the right disc (2) is an outer layer of the right disc (2), and a connecting method is an integral weaving and shaping connection or a split-type physical connection, the connecting wire (6) of the split-type physical connection is made into an independent structure and made of metal or a polymer wire material or a degradable polymer material, and then connected to the right disc (2).

5. The puncturable atrial septal occluder according to claim 4, wherein the split-type physical connection is specifically welding or braiding or suture.

## Patentansprüche

1. Punktierbarer Vorhofseptum-Okkluder, umfassend einen Stent, der durch Wärmeeinstellung hergestellt wird, nachdem ein Drahtmaterial mit einer Memory-Eigenschaft integral geflochten wurde, wobei der Stent aus einer linken Scheibe (1), einer rechten Scheibe (2), und einer Taille (3) besteht, die verbunden sind, wobei der Okkluder ferner ein mittleres Durchgangsloch (4), das den Stent durchdringt, und eine Kerbe umfasst, die an den Mitten der linken Scheibe (1) und der rechten Scheibe (2) gebildet ist, wobei die linke Scheibe (1), die rechte Scheibe (2) und die Taille (3) aus einer integralen netzförmigen Struktur mit einem Drahtmaterial als Memory-Legierungsmaterial bestehen, die linke Scheibe (1) und die rechte Scheibe (2) beide eine doppellagige netzförmige Struktur haben, die Ränder der Kerben an den Mitten der linken Scheibe (1) und der rechten Scheibe (2) beide dicht gewebt sind, eine Webdichte eines verstärkten Webbereichs 2-5 Mal so hoch ist wie die anderer gemeinsamer Bereiche, das mittlere Durchgangsloch (4) einen Durchmesser hat, der dem Innendurchmesser der Taille (3) weitgehend entspricht und so ausgebildet ist, dass eine Punktionshülle darin hindurchpassen kann, eine Trennmembran (5) in dem mittleren Durchgangsloch (4) vorgesehen ist, um das mittlere Durchgangsloch (4) von deren Innenseite aus zu blockieren; eine Kerbe in der Mitte der rechten Scheibe (2) mit einer Vielzahl von Verbindungsdrähten (6) versehen ist, ein Ende jedes der Vielzahl von Verbindungsdrähten (6) konvergiert und mit einer Ausgabekomponente (7) verbunden ist, und das andere Ende jedes der Vielzahl von Verbindungsdrähten (6) bezüglich der Mitte der rechten Scheibe (2) radial mit der rechten Scheibe (2) verbunden ist, **dadurch gekennzeichnet, dass** die Vielzahl von Verbindungsdrähten (6) drehbar und symmetrisch gegenüber einer Ausgabekomponente (7) und dem mittleren Durchgangsloch (4) verteilt sind, und zwischen den benachbarten Verbindungsdrähten ein Spalt vorgesehen ist, durch den der Punktionsmantelkanal hindurchgehen kann, eine Anzahl der Verbindungsdrähte (6) höchstens 12 und mindestens 3 beträgt, die Ausgabekomponente (7) als Drahtklemmstahlhülse oder als verdichtete Kugel ausgebildet ist, ein Ende der Ausgabekomponente (7) mit dem Verbindungsdraht verbunden ist, das andere Ende der Ausgabekomponente (7) ein freies Ende ist, und das freie Ende mit einem Anschlussport versehen ist und mit einem Ausgabesystem verbunden ist, sodass der punktierbare Vorhofseptum-Okkluder in eine Ausgabeschleuse eingezogen oder aus ihr herausgedrückt werden kann.

2. Punktierbarer Vorhofseptum-Okkluder nach Anspruch 1, wobei der Okkluder ein morphologisches Gedächtnis mit zwei Morphologien der Kontraktion und Expansion aufweist.

3. Punktierbarer Vorhofseptum-Okkluder nach Anspruch 1, wobei die Trennmembran (5) eine Folienform aufweist und aus einem Polymermaterial oder einem abbaubaren Material besteht und die folienförmige Morphologie der Trennmembran eine Querschnittsform aufweist, die der des mittleren Durchgangslochs (4) entspricht.

4. Punktierbarer Vorhofseptum-Okkluder nach Anspruch 1, wobei eine Verbindungsposition des Verbindungsdrahts (6) an der rechten Scheibe (2) eine äußere Schicht der rechten Scheibe (2) ist und ein Verbindungsverfahren eine integrale Web- und Formverbindung oder eine physikalische geteilte Verbindung ist, wobei der Verbindungsdraht (6) der physikalischen geteilten Verbindung zu einer unabhängigen Struktur hergestellt und aus Metall oder einem Polymerdrahtmaterial oder einem abbaubaren Polymermaterial hergestellt und dann mit der rechten Scheibe (2) verbunden ist.

5. Punktierbarer Vorhofseptum-Okkluder nach Anspruch 4, wobei die physikalische Verbindung vom Split-Typ speziell Schweißen oder Flechten oder Nähen ist.

## Revendications

1. Dispositif d'occlusion de septum auriculaire pouvant être perforé, comportant un stent réalisé par thermofixation après le tressage intégral d'un matériau filaire à propriété de mémoire, le stent étant constitué d'un disque gauche (1), d'un disque droit (2), et d'une taille (3) qui sont connectés, le dispositif d'occlusion comportant en outre un trou traversant central (4) qui pénètre dans le stent, et une encoche formée au niveau des centres du disque gauche (1) et du disque droit (2), dans lequel le disque gauche (1), le disque droit (2) et la taille (3) présentent une structure maillée intégrale avec un matériau filaire en tant que matériau d'alliage à mémoire, le disque gauche (1) et le disque droit (2) présentant tous deux une structure maillée à double couche, des bords d'encoches au niveau des centres du disque gauche (1) et du disque droit (2) sont tous deux densément tissés, une densité de tissage d'une zone de tissage renforcée est de 2 à 5 fois supérieure à celle d'autres zones communes, le trou traversant central (4) présente un diamètre proche d'un diamètre interne de la taille (3) dans la plus grande mesure et est configuré de sorte qu'une gaine de ponction soit capable de passer au travers, une membrane de déflecteur (5) est prévue à l'intérieur du trou traversant central (4) pour bloquer le trou traversant central (4) à partir de l'intérieur de celui-ci ; une encoche au niveau du centre du disque droit (2) est dotée d'une pluralité de fils de connexion (6), une extrémité de chacun de la pluralité de fils de connexion (6) est convergente et connectée à un composant d'administration (7), et l'autre extrémité de chacun de la pluralité des fils de connexion (6) est radialement connectée au disque droit (2) par rapport au centre du disque droit (2), **caractérisé en ce que** la pluralité des fils de connexion (6) est répartie de manière rotative et symétrique par rapport à un composant d'administration (7) et au trou traversant central (4), et un espace à travers lequel le canal de gainage de ponction peut passer est prévu entre les fils de connexion adjacents, un nombre des fils de connexion (6) est de 12 au plus, et d'au moins 3, le composant d'administration (7) est configuré sous la forme d'un manchon en acier de serrage de fil ou d'une bille condensée, une extrémité du composant d'administration (7) est connectée au fil de connexion, l'autre extrémité du composant d'administration (7) est une extrémité libre, et l'extrémité libre est dotée d'un port de connexion, et connectée à un système d'administration, de sorte que le dispositif d'occlusion de septum auriculaire pouvant être perforé puisse être tiré dans une gaine d'administration ou sorti de celle-ci.

2. Dispositif d'occlusion de septum auriculaire pouvant être perforé selon la revendication 1, dans lequel le dispositif d'occlusion présente une mémoire morphologique, avec deux morphologies de contraction et d'expansion.

3. Dispositif d'occlusion de septum auriculaire pouvant être perforé selon la revendication 1, dans lequel la membrane de déflecteur (5) présente une forme de feuille et est composée d'un matériau polymère ou d'un matériau dégradable, et la morphologie en forme de feuille de la membrane de déflecteur présente une forme de section transversale identique à celle du trou traversant central (4).

4. Dispositif d'occlusion de septum auriculaire pouvant être perforé selon la revendication 1, dans lequel une position de connexion du fil de connexion (6) sur le disque droit (2) est une couche externe du disque droit (2), et un procédé de connexion est une connexion de tissage et de mise en forme intégrale ou une connexion physique de type fendu, le fil de connexion (6) de la connexion physique de type fendu est réalisé sous la forme d'une structure indépendante et est composé de métal ou d'un matériau de fil polymère ou d'un matériau polymère dégradable, puis connecté au disque droit (2).

5. Dispositif d'occlusion de septum auriculaire pouvant être perforé selon la revendication 4, dans lequel la connexion physique de type fendu est spécifiquement une soudure ou une tresse ou une suture.
